Europäisches Patentamt

European Patent Office

Office européen des brevets

(11) Publication number: **0 293 598**
**A2**

# EUROPEAN PATENT APPLICATION

(21) Application number: 88106774.8

(22) Date of filing: 27.04.88

(51) Int. Cl.4: **A01H 1/02 , A01G 7/00 , C12N 5/00**

(30) Priority: 29.04.87 US 43864

(43) Date of publication of application:
07.12.88 Bulletin 88/49

(84) Designated Contracting States:
AT BE CH DE ES FR GB GR IT LI LU NL SE

(71) Applicant: **DNA PLANT TECHNOLOGY CORPORATION (under the laws of the state of Delaware)**
**2611 Branch Pike**
**Cinnaminson, New Jersey(US)**

Applicant: **HERSHEY FOODS CORPORATION**
**100 Mansion Road**
**Hershey, PA 17033(US)**

(72) Inventor: **Sondahl, Maro R.**
**138 Society Hill**
**Cherry Hill, New Jersey 08003(US)**
Inventor: **Sereduk, Thomas B.**
**52 Rockland Terrace**
**Mt. Holly, New Jersey 08060(US)**
Inventor: **Bellato, Claudia M.**
**Kings Highway, Towers C-207/1**
**Maple Shade, New Jersey 08052(US)**
Inventor: **Chen, Zhenghua**
**Building 804, Room No. 504**
**Zhong Guan Cun Nan Lu, Beijing(CN)**

(74) Representative: **Patentanwälte Grünecker, Kinkeldey, Stockmair & Partner**
**Maximilianstrasse 58**
**D-8000 München 22(DE)**

(54) Somatic embryogenesis and plant regeneration of cacao.

(57) The present invention relates to a method of controlled regeneration of cacao plant which comprises:

providing a somatic embryo;

culturing the somatic embryo on a maturation medium capable of inducing differentiation of the embryo and

culturing the differentiated embryo on a medium capable of inducing germination, to produce plantlets capable of developing into mature plants.

The invention also relates to a method of providing somatic embryos which comprises:

culturing mature somatic tissue on a primary culture medium to obtain immature embryos; and

culturing the immature embryos on an isolation medium capable of inducing completion of the development of somatic embryos.

Therefore, the invention provides a means by which plantlets and mature plants can be obtained from somatic embryos of any source, either maternal or zygotic. It also provides a means for obtaining somatic embryos from two new sources from adult donor plants, namely nucellus and young petals from young flower buds.

The methods according to the present invention allow the production of genetic carbon copies or clones of a known donor plant which has been selected under field conditions.

# SOMATIC EMBRYOGENESIS AND PLANT REGENERATION OF CACAO

## FIELD OF THE INVENTION

This invention relates to the field of improvement of agricultural crop species. More specifically, the invention provides a method for the induction of somatic embryogenesis in cacao and the regeneration of mature plantlets therefrom.

## BACKGROUND OF THE INVENTION

The perfection of plant regeneration capabilities and the application of biotechnological techniques for genome modification provide a highly desirable system for the improvement of crop species.

Investigations concerning the morphogenesis of plant tissue in culture date back at least to the 1950s (Skoog, F. and Miller, C. O. , Symp. Soc. Exp. Biol., 11:118 (1957)) and have continued apace to date. Several monographs provide extensive reviews of the field and contain compilations of numbers of species which will undergo plant regeneration in culture (see for example, Murashige, T., .In: "Propagation of Higher Plant Through Tissue Culture," T.A. Thorpe, ed., p. 15, Univ. Calgary Press, Calgary (1978); Vasil, I.K. et al., Adv, Genet. 20:127 (1979) and Evans, D.A., et al. In: "Plant Tissue Culture: Methods and Applications in Agriculture: T.A. Thorpe, ed., pg 45, Academic Press, New York (1981)).

The impressive list of plant species cited in the above-referenced monographs, for which successful regeneration has been achieved, belies the difficulties in achieving those results. As will be noted later, successful regeneration of a particular species is often characterized by the addition of (or even omission of) catalytic amounts of auxins, cytokinins, or other growth regulators. Further, successful regeneration may also be a function of not only the mere presence of a certain compound but its ratio to other media components as well. Since each plant species appears to possess a unique optimal set of media requirements, the successful preparation and regeneration of a new species cannot be necessarily inferred from the successful regimens applied to unrelated plant species.

Despite the recent advances in plant regeneration for a variety of species, cacao (Theobroma cacao) is one of the crops which has been refractory to regeneration protocols; hence, the application of plant cell culture for improvement of this crop has lagged behind that progress in the field in general.

Absent a functioning regeneration protocol, more traditional avenues for crop improvement have been utilized. One approach has been to introduce into the commercial cacao genome agronomically useful characteristics derived from exotic or "wild" Theobroma cacao germplasm by conventional sexual hybridization and backcrossing breeding procedures.

Initial investigation relating to the tissue culture of Theobroma cacao demonstrated that it was possible to obtain callus from somatic tissue with relative ease. In fact an early report (Archibald, J.F., Nature 173:351-352 (1954)) disclosed that it was possible to obtain callus from bark or stem explants on a White's basal culture medium without any growth regulators. Longer term sustained culture of the callus did, however, require a more complex medium. In retrospect the ease of callus formation has in fact been an obstacle rather than an advantage. This tendency toward unorganized growth in culture has heretofore frustrated attempts to successfully regenerate plants in this system since tissue organization in the form of organs and/or embryos is often a predicate to regeneration of plantlets.

Subsequently, attempts to culture terminal or axillary buds were reported by Orchard , J E. et. al., (Physiol. Plant 47:207-210 (1979)). Although some bud swelling and leaf elongation were reported, the vegetative propagation of intact plants by this method was unsuccessful.

Another approach has been described by E.B. Esan (In: Proceedings Fifth Int'l. Conf. on Cacao Research, Ibadan, Nigeria, pgs. 116-124 (1973)). According to these methods, somatic embryos can be obtained through budding of cotyledon and hypocotyl tissue of young sexual embryos. Attempts to recover complete plants from these adventitious embryos failed. Employing a somewhat similar approach, Janick et al. (U.S. Patents 4,204,366; 4,291,498; 4,301,619 and 4,545,147) disclose methods for the induction and propagation of somatic embryos of cacao. Wang and Janick (Hort. Science 19:839-841) also described precocious germination of cacao somatic embryos, but were unable to obtain mature plants from them. In each case the starting material used to initiate the embryogenesis protocol was zygotic embryo tissue. The disadvantage of techniques predicated upon the use of zygotic embryos is that since cacao is an out crossed species the immature embryos will be of unknown genotype, and thus inappropriate for cloning. Although Litz ("Tissue Culture Studies with Theobroma cacao, Proc. Symp. Cacao Biotech., P.S. Dimick (ed.), p. 111-120, 1986) does describe the development of somatic embryos from mature somatic (leaf) tissue, all attempts at obtaining plantlets from these embryos have also proven futile.

The present invention provides a means by which plantlets and mature plants can be obtained from somatic embryos of any source, either maternal or zygotic. It also provides a means for obtaining somatic embryos from two new sources from adult donor plants, namely, nucellus and young petals from young flower buds. As used herein, "young petals" and "young flower buds" are defined as petals derived from unopened buds, usually about 5-8 mm in length; the preferred petals are generally about 4-6 mm. The ability to regenerate plants from maternal somatic tissue is extremely useful in any form of breeding strategy in which knowledge of the genotype of progeny is critical to its success. For example, somatic embryogenesis from maternal somatic tissue, which ultimately results in plant production, is an important step in applying large scale vegetative propagation to selected donor plants in the field, and for development of selected parents for hybrid seed production. It is also a useful step in production of somaclones, in vitro selection of mutants, protoplast isolation, and DNA uptake via protoplasts or directly via microinjection or electroporation.

Somatic embryogenesis in cacao which had previously been described, as discussed above, relied largely on tissue from immature sexual embryos, which, because cacao is not self-pollinating, necessarily does not represent the genotype of the maternal donor plant alone; thus the genetic origin of the somatic embryos is also unknown. In the cases in which it has proven possible to provide somatic embryos from maternal somatic tissue, it was not possible to get past this stage. In no case has the production of a mature cacao plant been achieved by somatic embryogenesis. The advantage of the present methodology is that it is capable of producing genetic carbon copies, or clones, of a known donor plant which has been selected under field conditions, in light of its superior qualities.

BRIEF DESCRIPTION OF THE INVENTION

The present invention relates to a method of controlled regeneration of cacao plant which comprises:
    providing a somatic embryo;
    culturing the somatic embryo on a maturation medium capable of inducing differentiation of the embryo and
    culturing the differentiated embryo on a medium capable of inducing germination, to produce plantlets capable of developing into mature cacao plants.

The invention also relates to a method of providing somatic embryos which comprises:
    culturing mature somatic tissue on a primary culture medium to obtain immature embryos; and
    culturing the immature embryos on an isolation medium capable of inducing completion of the development of embryos.

The foregoing method is particularly well adapted to development of embryos from nucellus and young petal tissue.

DETAILED DESCRIPTION OF THE INVENTION

On their face, the principles underlying plant tissue culture are quite simple. Initially, it is necessary to isolate a plant part from the intact plant and disrupt its organ, inter-tissue, or inter-cellular relationships. Subsequently, it is necessary to provide the isolated material with the appropriate environment in which to express its intrinsic or induced developmental potential. Finally, all these steps must be carried out aseptically. Although the principles may be simply stated, as a matter of practice, the successful culture of plant tissue and its regeneration into a mature plant is extremely complex.

There are two general patterns of in vitro embryogenesis: direct initiation from differentiated tissue, and indirect initiation via a callus intermediary. Direct embryogenesis proceeds from embryogenically-determined cells. Indirect embryogenesis requires dedifferention of embryogenically-determined cells, callus proliferation, and differentiation of embryogenic cells.

Plant regeneration via a callus intermediary may be envisioned to comprise three stages. The first stage occurs following the transfer of an explant onto a culture medium. This stage is characterized by a proliferation of the explant or callus. The second stage is characterized by a rapid increase in embryo and organ growth. This stage may require a transfer to a second medium with or without a change in growth regulator concentration. The final stage occurs when the plants are removed from in vitro culture and requires the establishment of the autotrophic state.

A number of experimental parameters must be addressed during the regeneration protocol. For example, for a particular species the source of the explant may be important for the success of the subsequent regeneration. The size and the shape of the explant may also be critical. Another element to be considered is the method of providing aseptic explant material for purpose of callus formation. This involves sterilization of the explant tissue prior to inoculation onto propagation medium. Even this apparently routine process is subject to a wide variety of critical experimental parameters.

The present invention has succeeded in producing cacao plantlets and mature plants by virtue of the recognition that the cacao embryo requires a maturation phase on a distinct medium. Several previous methods have attempted regeneration by continued culturing of immature embryos on a primary culture medium. In fact, callus is extremely easy to develop from a variety of different organs or explants, or a wide range of culture medium. However, obtaining any further substantial differentiation, i.e., past the somatic embryo stage, has proven difficult, if not impossible. Similarly, as in the case of Wang and Janick, cited supra, it was possible to initiate precocious germination in somatic embryos, but still impossible to develop any actual plantlets from the germinated seedling. It has now been discovered to be essential that the embryos, regardless of the source, must be passed through this maturation phase in order to ultimately be capable of producing viable plantlets. Passage through this maturation phase appears to be critical in obtaining normal adult plants from somatic embryos, the distinct treatment being necessary to the normal differentiation of the embryo, i.e., root and shoot tip formation.

The morphologically fully developed embryo can be readily recognized by its distinct form, wherein complete hypocotyl and cotyledonary leaves are present. At this stage, the embryos should be transferred to the maturation medium. The essential components of an adequate maturation medium appears to include the presence of at least one cytokinin and preferably two cytokinins; when two are employed, it is preferred that one is natural and one is synthetic. The natural cytokinin is useful in promoting cell division generally, whereas the synthetic cytokinins are useful in inducing shoot tip formation. Among the natural cytokinins which may be used are zeatin or kinetin, while synthetic cytokinins are preferably selected from 6-BA (benzyl adenine) or 2-iP (isopentyl adenine). The synthetic cytokinin is typically present in an amount which is about twice that of the natural cytokinin, i.e., about 2-5 uM, compared with about 1-2.5 uM for the natural components.

Also required during the maturation phase is at least one auxin. A number of different types of auxin are available, both natural and synthetic; among these are indole-3-proprionic acid, indole-3-butyric acid, indole-pyruvic acid, phenylacetic acid, phenoxyacetic acid, naphthoxyacetic acid, naphthalene acetic acid, and indole acetic acid. The preferred auxins in the present case are naphthylene acetic acid (NAA) and indole acetic acid (IAA). At least one of these compounds must be present in the maturation medium, in an amount of about 0.5-2.0 uM total.

Another fundamental element of the maturation medium is gibberellic acid (GA), preferably present in an amount of about 1-2 uM. Abscisic acid is also a preferred component and, when used, is present in an amount of about .01-.07 uM.

In addition to the growth regulators, the presence of sucrose or an equivalent sugar as osmoticum as well as a nutrient source in the medium is also important. When sucrose is employed, the amount is generally about 80-140 g/l of medium. This provides an osmoticum equivalent in the range of -5.8 bars to -10.1 bars. However, it is also possible to substitute other sugars, such as glucose, mannitol or sorbitol to obtain equivalent effects.

The foregoing elements are those which are critical to the successful initiation of differentiation of the embryo. However, the maturation medium will of course contain a number of elements which, while necessary for the normal growth and metabolism of the embryo, do not themselves affect maturation. Obviously, it is necessary to have at least one exogenous nitrogen and carbon source, as well as desirable to have the preferred vitamins and salts, but the nature of the elements is not particularly critical.

For example, the basal medium is preferably Murashige and Skoog (MS) medium having a specific formulation of macro- and micronutrients, which formulations are well known in the art (see, e.g., Handbook of Plant Cell Culture, Evans et al., Vol. I, 1984). The particular concentration of the salts at this phase is not critical, but generally the macronutrients will typically be used in an amount of about 0.25-0.5 X, and micronutrients at about 0.5-1.0 X. The presence of additional iron salts, such as MSV (Murashige and Skoog iron solution), are also important and preferably are added in an amount of about 0.5-2 X. Nitrogen sources may be provided by addition of individual amino acids, in the form of a protein hydrolysate such as casein hydrolysate (casein HCl), or both. Various sources of carbon, other than the sugar used as osmoticum, may also preferably be added; a particularly useful source of a variety of complex sugars is malt extract, which also contains vitamins, minerals, amino acids, and hormones. Any convenient source of vitamins may also be employed. Agar may be added, to solidify the medium, or the medium may be liquid, as may the subsequently used medium, and the pH is adjusted to about 4-5.5. Low light, i.e, from 300-1000 lux, is preferred, with 300-400 lux being most preferred. Embryos are allowed to remain on the maturation medium for about 30-60 days, with 30-35 days being the optimal time period for maturation, before transfer to a germination medium. A visual indicator for the time of transfer is the development on the embryo of a perceptible swelling at the site of the apical meristem, indicating shoot tip development. This can generally be readily observed with the aid of a low power magnifying glass or microscope. Root tip development is also helpful in gauging the time of transfer but is not as critical as shoot tip development. Particularly good results are observed when there is a serial transfer throughout the time period from a medium which starts out with lower levels of the synthetic cytokinin to a medium with higher levels of cytokinin; this is demonstrated in Example 1. However, this progressive transfer is by no means critical to the success of the maturation stage.

As noted above, it is this maintain phase treatment which is actually the key to obtaining mature plants in the present method. Germination may be obtained in any medium having the appropriate concentration of growth regulators. The germination medium should contain at least one cytokinin, in an amount of about 2-5 uM; also, a natural auxin, e.g. IAA, should be present, at about 0.25-1 uM. As in maturation, GA and ABA should be included, at about 1-2 uM and .01-.07 uM, respectively. There is no longer a necessity for sucrose to be present in large amounts as osmoticum, although small amounts usually no more than 4%, can remain as a source of energy. The remaining composition of medium is essentially the same as described for the maturation medium, i.e., a general mixture of nitrogen, carbon, and vitamin sources, with less than full strength (i.e., 1X) MS salts. The pH of the medium is preferably about 5-6. Light conditions are generally high, about 1000-3000 lux, with about 2500 being preferred. Germination normally occurs in about 20-35 days, with 20 days being the optimal germination period. After germination, plantlets are transferred to soil in pots and allowed to mature. As used in the present specification and claims, a mature plant is understood to mean one which does not possess any typical juvenile characteristics and which is capable of producing fruits and flowers. The foregoing procedure has been successful in producing mature plants from embryos of a variety of different cacao genotypes. Although the optimal composition of the medium may differ for each genotype, the general compositions outlined above routinely produce mature plants to a greater or lesser extent. The minor modifications of the nonessential components of the medium which may be necessary to optimize yield are well within the skill of the experienced worker, without the necessity for undue experimentation.

As already noted, the present method can be employed to obtain whole plants from somatic embryos from any source, although, of course, embryos derived from mature somatic tissue is preferred. In this vein, the present invention also provides a novel method for obtaining somatic embryos from mature tissues. The present method has proven useful in the development of somatic embryos from two new sources of mature tissue, namely nucellus and young flower bud petals. "Nucellus" refers to the tissue in an ovule, in which the embryo sac develops, and young flower bud petals refers to the flower bud including the flower petals. This method has proven particularly useful in combination with the foregoing maturation and germination procedures for regenerating mature cacao plants However, the procedure may also be used in any situation in which production of somatic cacao embryos are expected to be useful.

In the present method two stages characterize the successful practice of the invention. The initial or inoculation stage does not differ substantially from the methods used in the art for development of embryos from zygotic tissue. The development of immature embryos from callus tissue is obtained by culturing explants of nucellus, or young petals, in a primary medium containing at least one synthetic auxin in an amount of about 2.5-15.0 uM, and at least one cytokinin, preferably selected from 2-iP, zeatin or kinetin, preferably in an amount of about 2.5 uM. The use of 2,4-D as auxin at this stage is strongly preferred. The medium is also supplemented with coconut water, an element frequently used in the art in embryogenic medium, in an amount of about 50-100 ml/l of medium. The remaining components do not differ significantly from the basic elements described previously for support of general growth and development: basal inorganic salts, preferably MS, at less than full strength, generally about 0.5 X; a source of nitrogen, such as casein hydrolysate and/or individual amino acids; a carbon source, which may be a simple sugar such as sucrose, or may be a complex source of sugars, such as malt extract; and any of the common sources of vitamins. The medium is solidified with agar, or other gelling agent, and the cultures maintained in the dark at a temperature of about 24-28°C for about 45 days, or until immature embryos, typically globular or heart-shaped, appear in the callus mass.

At this point, immature embryos must be transferred to a second isolation and development medium. The presence of specific growth regulators in this medium is essential to the successful completion of embryo development. The development medium must contain at least one cytokinin, in a to the amount of about 0.5-4 uM. The preferred cytokinin is zeatin, but this may also be combined with kinetin or another cytokinin. However, 6BA is not particularly recommended for use at this stage. The presence of gibberellic acid and ABA is also critical, in concentrations of about 0.3-1 uM and 0.01-0.07 uM, respectively.

The remainder of the medium composition is, as with the inoculation medium, not restricted to any particular composition. Again MS salts are preferred with macroelements preferably being present in an amount of about 0.25-0.5 X and microelements at 0.5-1.0 X. Additional sources of vitamins, nitrogen and carbon are also typically present. Coconut water may be used to provide a convenient source of many of these elements. Sucrose is the preferred carbon source, in an amount of about of about 25-40 g/l; however, sucrose may be replaced by similar quantities of glucose or a 1:1 mixture of fructose to glucose. The pH is preferably maintained in a range of about 5-6. The embryos are incubated on the solidified medium at a temperature of about 24-28°C, under low light (300-1000 lux, preferably 300-400) conditions for a period of about 25-40 days; a 30-day period appears to be optimal. As an optional element in this stage, the developing embryos may be periodically transferred to new media with a progressive increase in the amounts of cytokinin and GA in the new medium. Whichever procedure is followed, at the end of the development stage, the embryos are fully matured and are ready to be transferred to a maturation medium, or to be used in any other procedures for which somatic embryos would prove useful.

Specific demonstration of application of both the somatic embryogenesis procedure and the further development of mature plants from somatic embryos, are presented in the following non-limiting examples.

EXAMPLE 1

PROTOCOL FOR SOMATIC EMBRYOGENESIS, EMBRYO GERMINATION, PLANTLET DEVELOPMENT, AND HARDENING

Young cacao fruits measuring 60-100 mm long have yielded the best nucellus explants for callus induction and regeneration. Fruits were split in half after surface sterilization with 80% ethanol and 2.1% sodium hypochlorite. Nucelli were isolated from immature seeds, cut in small segments, and placed on primary culture medium. The basal end of the nucellus was always eliminated to avoid the presence of sexual embryo tissues.

There are several steps or phases that characterize the successful practice of this invention.

Phase 1 - Inoculation and Regeneration

Segments of nucellus tissue were inoculated on primary culture medium. The composition of this medium is as follows: half concentration of Murashige and skoog (MS) inorganic salts, thiamine (30 uM), pyridoxine (15 uM), nicotinic acid (15 uM), glycine (5 mg/l), cysteine (200 mg/l), casein hydrolysate (50-100 mg/l), malt extract (100-500 mg/l), polyvinylpyrrollidone (PVP) (5-10 g/l), coconut water (50-100 ml/l), sucrose (20-40 g/l), 2,4-Dichlorophenoxy acetic acid (0.5-3.0 mg/l), 2-iP (0.5 uM), agar (7 g/l) or gelrite (2.3 g/l), pH 5.3. The medium is then autoclaved. Primary cultures were maintained at 24-28°C temperature under dark conditions.

Phase 2 - Isolation and Emtryo Development

Early stages of somatic embryos were isolated from callus mass and explant pieces onto a secondary embryo development medium under light conditions. This isolation and transfer to different media permitted the completion of embryo development. More than one culture medium was used in succession to nurse embryo development. Details of the culture media used during this phase are presented in Table I.

Phase 3 - Maturation

After asexual cacao embryos complete their developmental phase (complete hypocotyl and cotyledons are present), they need to undergo a maturation step in order to produce a shoot and root pole before germination is permitted. A series of successive transfers to different culture media (under light conditions) were used during this phase (see Table I for the various culture media employed).

Phase 4 - Germination

Somatic embryos with a prominent shoot and root pole were transferred onto a germination medium (see Table I for germination media) to further develop primary leaves and roots. At completion of this step, young cacao plantlets are obtained.

Phase 5 - Plantlet Development

All culture Phases 1-4 were conducted on solidified agar or gelrite medium. At this step, cacao plantlets were removed from the solid medium, washed in sterile water, treated with rooting hormones (IBA or NAA 200-600 ppm or a mixture of both), and inoculated in sterile soil mix containing half strength MS salts and Zeatin (0.5-2 uM).

## Phase 6 - Hardening In A Greenhouse

Plantlets with 2-4 primary leaves and an acceptable root system (primary and secondary roots) were transferred from laboratory growth chambers to shaded areas of a greenhouse (20% sunlight). Before moving these cacao plantlets from the laboratory to the greenhouse, they were immersed in a fungicide solution and then transferred to small pots (3-inch size) or growing cones (10 cu inches) containing the same soil mix and nutrients described in Phase 5. Each plantlet was covered with a PVC film to protect against excess dehydration. During the first 1-2 weeks, small holes were made in these PVC covers and later these PVC film were removed completely. The young cacao plants derived from somatic embryos were kept under special section of greenhouse with a fog system until they were transferred to one-gallon pots.

EP 0 293 598 A2

## TABLE I

### CULTURE MEDIA COMPOSITIONS FOR CACAO EMBRYOGENESIS

| Component | ISOLATION | | | MATURATION | | | | GERMINATION | |
|---|---|---|---|---|---|---|---|---|---|
| | SEVIii | 4G | 4Gi/4 | 4P/13 | 4P/14 | 4P/14B | 4P/15 | 4P/16 | 15C/6 |
| Macroelements | 0.25X | 0.50X | 0.50X | 0.50X | 0.50X | 0.50X | 0.50X | 0.50X | 0.50X |
| Microelements | 0.25X | 0.0X | 1.0X | 1.0X | 0.50X | 0.50X | 0.50X | 0.50X | 0.50X |
| Iron Salt (MSV) | 0.25X | 1.0X | 1.0X | 1.0X | 1.0X | 1.0X | 1.0X | 1.0X | 1.0X |
| Vitamins | 1.0X | 1.0X | 1.0X | 1.0X | 1.0X | 1.0X | 1.0X | 1.0X | 0.50X |
| Inositol (mg/l) | 100 | 100 | 100 | 100 | 100 | 100 | 100 | 100 | 0 |
| $VB_2$ (mg/l) | 0 | 0.1 | 0.05 | 0.1 | 0.1 | 0.1 | 0.1 | 0.1 | 0.1 |
| Cysteine (mg/l) | 200 | 200 | 200 | 200 | 200 | 200 | 200 | 200 | 0 |
| Glycine (mg/l) | 5 | 5 | 5 | 5 | 5 | 5 | 5 | 5 | 0 |
| Caseine HCl (mg/l) | 0 | 0 | 0 | 500 | 500 | 500 | 100 | 100 | 0 |
| Malt Extract (mg/l) | 100 | 100 | 100 | 100 | 100 | 100 | 100 | 100 | 0 |
| CW (ml/l) | 50 | 50 | 50 | 0 | 0 | 0 | 25 | 25 | 25 |
| 6BA (uM) | 0 | 0 | 0 | 2.5 | 5.0 | 5.0 | 5 | 5 | 5 |
| ZEA (uM) | 1 | 2.5 | 2.5 | 2.5 | 2.0 | 2.0 | 2 | 2 | 0 |
| KIN (uM) | 0 | 0 | 1.5 | 0 | 0 | 0 | 0 | 0 | 0 |
| NAA (uM) | 0 | 1 | 0 | 1.5 | 1.5 | 1.5 | 1.5 | 0 | 0 |
| IAA (uM) | 0 | 0 | 0 | 0 | 0 | 0 | 0 | 1.0 | 1.0 |
| GA (uM) | 0.3 | 1 | 1 | 1.5 | 1.5 | 1.5 | 1.5 | 1.5 | 1.5 |
| ABA (uM) | 0.05 | 0.05 | 0.05 | 0.02 | 0.02 | 0.02 | 0.02 | 0.02 | 0.02 |
| PVP (g/l) | 5 | 5 | 5 | 5 | 5 | 5 | 5 | 5 | 0 |
| Charcoal (g/l) | 0 | 0 | 0 | 2 | 5 | 2 | 2 | 5 | 5 |
| Sucrose (g/l) | 30 | 40 | 40 | 80 | 80 | 80 | 80 | 80 | 40 |
| Agar (g/l) | 9 | 9 | 9 | 8.5 | 8.0 | 8.0 | 8.0 | 8.0 | 8.0 |
| pH | 5.3 | 5.3 | 5.3 | 4.0 | 4.0 | 4.0 | 5.3 | 5.3 | 5.3 |
| Light Intensity | Low | Low | Low | Low | Low | Low | High | High | High |

EXAMPLE 2

## PROTOCOL FOR SOMATIC EMBRYOGENESIS FROM IMMATURE PETAL TISSUES

Young flower buds were excised from adult cacao plants and surface sterilized in 0.8% sodium hypochlorite for 10 minutes. Young flower bud petals were isolated with dissecting needles under steroscope using sterile glass slides. Immediately after isolation, all young petals were transferred onto the following primary medium: MS inorganic salts, inositol (550 uM), thiamine (30 uM), pyridoxine (15 uM), nicotinic acid (15 uM), 2,4-D (10 uM), kinetin (1 uM), sucrose (40 gl/l), pH 5.5, autoclaved. Alternatively, the following primary medium can be used: half strength MS inorganic salts, thiamine (30 uM), pyridoxine (15 uM), nicotinic acid (15 uM), inositol (550 uM), casein HCl (500 mg/l), glycine (5 mg/l), cysteine (250 mg/l), PVP (5 g/l), malt extract (500 mg/l), sucrose (60 g/l), coconut water (100 m/l), 2,4-D (15 mg/l), 2iP (2.5 uM), pH 5.3, autoclaved. Petals from 6 mm long flower buds exhibited the best response, however, buds of from about 4 mm to about 8 mm can be employed. A pretreatment at 10° C for 24 hours before inoculation onto the primary medium were beneficial for regeneration. The cultures were maintained at 23-25° C under dark conditions.

After 3 weeks on primary medium, direct embryogenesis were observed on the basal portion of young petal explants. Callus formation was observed on the distal end of young petals. Tissues containing early stage of somatic embryos (globular to heart shape) were transferred to the following liquid medium: MS inorganic salts, coconut water (100 ml/l), sucrose (50 g/l), pH 5.3. A genotype dependent response was noted, whereby germplasm H-4 and H-13 were the most responsive relative to three others tested.

Once the young somatic embryos derived from immature petals were removed from the original tissues, they were cultivated in culture media already described for nucellus-derived embryos, i.e. media of Phases 2, 3, 4, 5 and 6. The culture conditions and physical parameters were the same as the ones used for nucellus embryos.

## Claims

1. A method for controlled regeneration of cacao plants which comprise

providing a somatic embryo;

culturing the somatic embryo on a maturation medium capable of inducing differentiation of the embryo, and

germinating the differentiated embryo on a germination medium to produce plantlets capable of developing into mature cacao plants.

2. The method of claim 1 wherein the maturation medium comprises at least one cytokinin, at least one auxin and gibberellic acid.

3. The method of claim 1 or 2 wherein the maturation medium comprises effective amounts of at least two cytokinins, at least one auxin, gibberellic acid, and abscisic acid.

4. The method of any of claims 1-3 wherein one cytokinin is natural, and one is synthetic.

5. The method of any of claims 1-4 wherein the natural cytokinin is zeatin or kinetin, and the synthetic cytokinin is 6-BA or 2-iP.

6. The method of any of claims 1-5 wherein the auxin is NAA or IAA.

7. The method of any of claims 1-6 wherein the medium comprises effective amounts of zeatin and 6-BA, NAA, gibberellic acid and abscisic acid.

8. The method of any of claims 1-7 wherein the synthetic cytokinin is present in an amount of about 2-5 uM, the natural cytokinin in an amount of about 1-2.5 uM, auxin in an amount of about 0.5-2.0 uM, gibberellic acid amount of about 1-2 uM, and abscisic acid at about 0.01-0.07 uM.

9. The method of claim 8 wherein the cytokinins are 6-BA and zeatin, and the auxin is NAA.

10. The method of any of claims 1-9 wherein the embryo is cultured in maturation medium for a period of about 30-60 days.

11. The method of any of claims 1-10 wherein the germination medium comprises effective amounts of at least one cytokinin, at least one natural auxin, gibberellic acid and abscisic acid.

12. The method of any of claims 1-11 wherein the germination medium comprises effective amounts of 6-BA, IAA, gibberellic acid and abscisic acid.

13. The method of any of claims 1-12 wherein the germination medium comprises cytokinin in an amount of about 2-5 uM; auxin in an amount of about .25-1 uM and abscisic acid in an amount of about 0.01-0.07 uM.

14. A method of obtaining somatic embryos from mature somatic tissue of cacao which comprises

culturing mature somatic tissue on a primary culture medium to obtain immature embryos, and

culturing the immature embryos in an isolation medium capable of inducing completion of the development of somatic embryos.

15. The method of claim 14 wherein the mature tissue is nucellus or young petals.

16. The method of claim 14 or 15 wherein the primary culture medium comprises effective amounts of at least one synthetic auxin and at least one cytokinin.

17. The method of any of claims 14-16 wherein the auxin is 2,4-D and the cytokinin is 2-iP, zeatin or kinetin.

18. The method of any of claims 14-17 wherein the auxin is present in an amount of about 2.5-15.0 uM and the cytokinin is present in an amount of about 2.5 uM.

19. The method of any of claims 14-18 wherein the isolation medium comprises effective amounts of at least one cytokinin, gibberellic acid, and abscisic acid.

20. The method of any of claims 14-19 wherein the isolation medium contains the cytokinin is zeatin or kinetin.

21. The method of any of claims 14-20 wherein the isolation medium contains cytokinin in an amount of about 0.5-4 uM, gibberellic acid in an amount of about 0.3-1 uM, and abscisic acid in an amount of about 0.01-0.07 uM.

22. The method of any of claims 14-21 wherein the embryo is cultured on isolation medium for about 25-40 days.

23. A method of controlled regeneration of cacao plants which comprises

culturing mature somatic tissue on a primary culture medium to obtain immature embryos;

culturing the immature embryos in an isolation medium capable of inducing completion of development of somatic embryos;

germinating the differentiated embryo on a germination medium, to obtain plantlets capable of developing into mature cacao plants.

24. The method of claim 23 wherein the somatic tissue is nucellus or young petals.

25. A mature cacao plant produced by the method of any of claims 1-13 or 23-24.

26. A somatic embryo produced by the method of any of claims 14-21.